# EUROPEAN PATENT APPLICATION

(11) **EP 2 255 783 A2**
(43) Date of publication of application: **01.12.2010**
(21) Application number: 10164060.5
(22) Date of filing: 27.05.2010
(51) Int. Cl.: A61K 9/06, A61K 9/127, A61K 31/20, A61K 8/362, A61Q 19/00

(54) **Azelaic acid gel, and a method of obtaining same**

(30) Priority: 28.05.2009 PL 38813509
(71) Applicant: Przedsiebiorstwo Produkcji Farmaceutycznej Hasco-Lek S.A., 51-131 Wroclaw (PL)
(72) Inventor: Langner, Marek, 51-314, Wroclaw (PL); Potaczek, Piotr, 58-508, Jelenia Gora (PL)
(74) Representative: Krekora, Magdalena

(57) **Abstract**

An azelaic acid gel consisting of azelaic acid, liposomes, organic solvents, buffer, gelling agents, and preferably additionally consisting of preservatives and antioxidants. The overall concentration of the azelaic acid is 15% by weight. Liposomes are obtained from purified soya or egg phosphatidylcholine or from 1,2-dioleoyl-sn-glycero-3-phosphatidylcholine. The organic solvents are propylene glycol, ethanol and triethanolamine. A method of obtaining the said composition is also disclosed.

## Description

### TECHNICAL FIELD

The object of the present invention is a gel comprising azelaic acid as active substance used for medical or cosmetic purposes, and a method of obtaining an azelaic acid gel.

### BACKGROUND ART

European patent EP0336880 describes pharmaceutical composition containing azelaic acid in concentration of 20% and excipients such as: triacylglycerols, and diacylglycerols, propylene glycol, polysorbate, water, and salt. These compositions are in the form of ointment, which is used for treatment of skin ageing.

In *Rote Liste* from 1996 under the number 32 282 a pharmaceutical composition named *Skinoren* may be found. This composition comprising azelaic acid is in the form of ointment, and is used for treatment of simple acne.

Polish patent PL202454 describes pharmaceutical composition in the form of hydrogel, comprising azelaic acid as active substance, and following excipients: triacylglycerol, propylene glycol, polyacrylic acid, soya lecithin, polisorbate, water and salt. Content of azelaic acid in pharmaceutical composition described in the patent is between 5-20% by weight. The most preferable composition of the pharmaceutical formulation described in this patent is a formulation of 14-16% of azelaic acid comprising not less than 1% by weight of soya lecithin. It is used in treatment of acne rosacea, senile skin, melanoderm, or/and skin irritation. This composition, comprising 15% by weight of azelaic acid is sold on the market under the trademark *Skinoren Gel* or *Finacea.*

### DISCLOSURE OF THE INVENTION

The objective of the present invention is to provide a gel for medical or cosmetic purposes comprising azelaic acid as active substance, which has stability required for such preparations, and at the same time increased accumulation in skin, what results in decrease of active substance content.

The invention describes an azelaic acid gel characterised in that it consists of azelaic acid, liposomes, organic solvents, buffer, gelling agents, and may additionally consist of preservatives and antioxidants. Preferably the overall concentration of the azelaic acid is 15% by weight. Liposomes, being a part of the gel composition, may be obtained from purified phosphatidylcholine, preferably from soya phosphatidylcholine or egg phosphatidylcholine. Preferably the overall concentration of purified phosphatidylcholine is from 10 to 15% by weight. Instead of purified phosphatidylcholine, purified 1,2-dioleoyl-sn-glycero-3-phosphatidylcholine is used as the source of liposomes. Preferably the overall concentration of 1,2-dioleoyl-sn-glycero-3- phosphatidylcholine is from 10 to 15% by weight. The azelaic acid gel may comprise monolayer liposomes or multilayer liposomes or a mixture of monolayer liposomes and multilayer liposomes. The azelaic acid comprised in the gel may be in the form not bound to liposomes or may be in the form bound to liposomes or in the form both bound and not bound to liposomes. Preferably the organic solvents are propylene glycol, ethanol and triethanolamine. Preferably the overall concentration of propylene glycol is from 6 to 10% by weight, the overall concentration of ethanol is from 3 to 5% by weight, the overall concentration of triethanolamine is from 7 to 7.5% by weight. The buffer comprised in the azelaic gel may be citrate buffer, preferably having the pH between 4.5 and 5.5. In such pH value amiphatic form of azelaic acid is generated resulting in maximum interaction between azelaic acid, and liposomes, and increase of azelaic acid solubility in the mixture. Preferably the antioxidant is disodium edetate, and its overall concentration is 0.5% by weight. Preferably the preservative is methyl parahydroxybenzoate, and its overall concentration is 0.15% by weight. Preferably the gelling agent is polyacrylic acid, and its overall concentration is from 1 to 3% by weight. Instead of polyacrylic acid, ammonium acryloyldimethyltaurate/VP copolymer may be used. Preferable overall concentration of ammonium acryloyldimethyltaurate/VP copolymer is from 1 to 3% by weight.

A method of obtaining the azelaic acid gel consisting of azelaic acid, liposomes, organic solvents, buffer, gelling agents, and preservatives and/or antioxidants, described in the invention, is characterised in that purified phosphatidylcholine or 1,2-dioleoyl-sn-glycero-3-phosphatidylcholine is dissolved in propylene glycol, and then mixed with aqueous phase comprising buffer, remaining organic solvents, antioxidant, preservative and azelaic acid to obtain a suspension of liposomes, which is then mixed with remaining part of azelaic acic and gelling substance. Preferably the suspension of liposomes is obtained by extrusion or by homogenisation.

Use of liposomes in the topical formulation in accordance with the present invention increases the concentration of the active substance directing it into the skin, and additionally used liposomes create thin phospholipid layer protecting the ointment against drying too quickly, and prolonging the bioavailability of azelaic acid.

Use of appropriate mixture of organic solvents for azelaic acid additionally increases bioavailability, what enhances efficacy of liposome formulation of azelaic acid.

Gels obtained in accordance with the examples given below underwent standard test in Franz cells in order to confirm their efficacy. Tests were carried out on pigs ears, whose impedance has been earlier measured, at the temperature of 32°C for 20 hours, when using phosphate buffer of pH equal to 7.4 in receiving chamber. Each preparation was tested in nine Franz cells.

Results from efficacy tests of liposome skin formulations in Franz cells, when pointing out the average concentration of azelaic acid on the skin, are shown at Diagram 1.

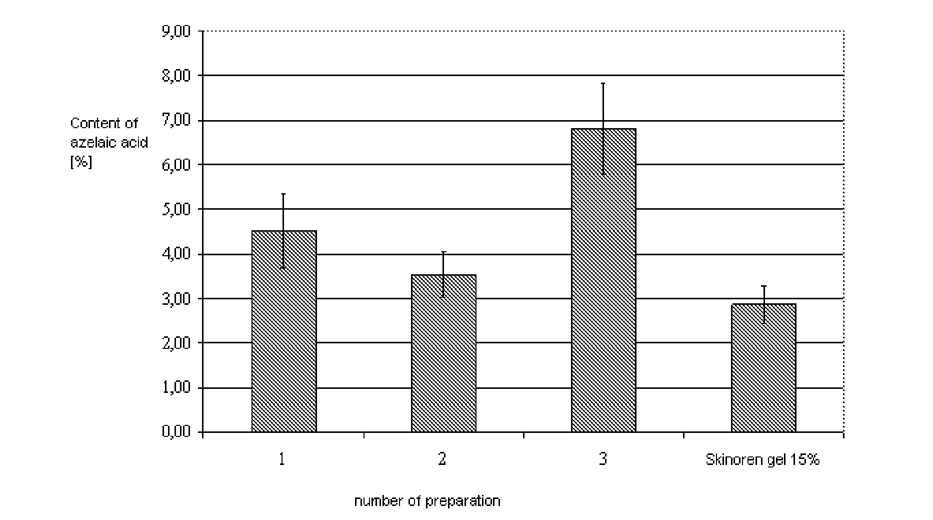

### BEST MODE OF CARRYING OUT THE INVENTION

The following examples illustrate the invention without setting or delineating its limits.

### Example 1.

Monohydrated citric acid, and sodium hydroxide are dissolved at room temperature in purified water receiving citrate buffer having pH equal to 5.0.

Ethanol is diluted with citrate buffer to concentration of 50%.

To the agitator tank equipped with stirrer, and heating jacket are added: azelaic acid (5%), 50% ethanol solution, citrate buffer of pH equal to 5, triethanolamine, disodium edetate, and methyl parahydroxybenzoate. The entirety is mixed at temperature of about 40°C until complete dissolution of all components.

Purified soya phosphatidylcholine is dissolved, when mixing, in propylene glycol at the temperature of about 70°C. To the so obtained solution is added by portions with continuous mixing, when deaerating, the solution of azelaic acid obtained earlier. After thorough deaeration the whole mixture is extruded once through two filters of 100 nm. To the liposome suspension is added remaining part of azelaic acid (10%). The entirety is mixed and deaerated simultaneously. Finally to the earlier obtained liposome suspension of azelaic acid is added ammonium acryloyldimethyltaurate/VP copolymer, and afterwards the entirety is thoroughly mixed and deaerated.

| *ACTIVE SUBSTANCES:* | |
|---|---|
| Azelaic acid | 15,00 |

| *EXCIPIENTS:* | |
|---|---|
| Purified soya phosphatidylcholine | 10,00 |
| Propylene glycol | 10,00 |
| Triethanolamine | 5,00 |
| Ethanol | 5,00 |
| Disodium edetate | 0,50 |
| Methyl parahydroxybenzoate | 0,15 |
| Ammonium acryloyldimethyltaurate/VP copolymer | 3,00 |
| Sodium hydroxide | 0,41 |
| Monohydrated citric acid | 1,02 |
| Purified water | 49,92 |
| TOTAL | 100,00 |

### Example 2.

Monohydrated citric acid, and sodium hydroxide are dissolved at room temperature in purified water receiving citrate buffer having pH equal to 5.0.

Ethanol is diluted with citrate buffer to concentration of 50%.

To the agitator tank equipped with stirrer, and heating jacket are added: azelaic acid (7.5%), 50% ethanol solution, citrate buffer of pH equal to 5, triethanolamine, disodium edetate, and methyl parahydroxybenzoate. The entirety is mixed at temperature of about 40°C until complete dissolution of all components.

Purified soya phosphatidylcholine is dissolved, when mixing, in propylene glycol at the temperature of about 70°C. To the so obtained solution is added by portions with continuous mixing, when deaerating, the solution of azelaic acid obtained earlier. After thorough deaeration the whole mixture is extruded once through two filters of 100 nm. To the liposome suspension is added remaining part of azelaic acid (7.5%). The entirety is mixed and deaerated simultaneously. Finally to the earlier obtained liposome suspension of azelaic acid is added ammonium acryloyldimethyltaurate/VP copolymer, and afterwards the entirety is thoroughly mixed and deaerated.

| *ACTIVE SUBSTANCES:* | |
|---|---|
| Azelaic acid | 15,00 |
| *EXCIPIENTS:* | |
| Purified soya phosphatidylcholine | 15,00 |
| Propylene glycol | 10,00 |
| Triethanolamine | 7,50 |
| Ethanol | 5,00 |
| Disodium edetate | 0,50 |
| Methyl parahydroxybenzoate | 0,15 |
| Ammonium acryloyldimethyltaurate/VP copolymer | 1,00 |
| Sodium hydroxide | 0,36 |
| Monohydrated citric acid | 0,92 |
| Purified water | 44,57 |
| TOTAL | 100,00 |

### Example 3.

Monohydrated citric acid, and sodium hydroxide are dissolved at room temperature in purified water receiving citrate buffer having pH equal to 5.0.

Ethanol is diluted with citrate buffer to concentration of 50%.

To the agitator tank equipped with stirrer, and heating jacket are added: azelaic acid (5%), 50% ethanol solution, citrate buffer of pH equal to 5, triethanolamine, disodium edetate, and methyl parahydroxybenzoate. The entirety is mixed at temperature of about 40°C until complete dissolution of all components.

Purified soya phosphatidylcholine is dissolved, when mixing, in propylene glycol at the temperature of about 70°C. To the so obtained solution is added by portions with continuous mixing, when deaerating, the solution of azelaic acid obtained earlier. After thorough deaeration the whole mixture is extruded once through two filters of 100 nm. To the liposome suspension is added remaining part of azelaic acid (10%). The entirety is mixed and deaerated simultaneously. Carbomer 941 is dispergated in water. Then 30% solution of sodium hydroxide is added in order to gel the polymer. The entirety is mixed and deaerated simultaneously. To such obtained gel is added by portions the liposome suspension of azelaic acid obtained earlier. The entirety is mixed and deaerated simultaneously.

| *ACTIVE SUBSTANCES:* | |
|---|---|
| Azelaic acid | 15,00 |

| *EXCIPIENTS:* | |
|---|---|
| Purified soya phosphatidylcholine | 10,00 |
| Propylene glycol | 6,67 |
| Triethanolamine | 5,00 |
| Ethanol | 3,33 |
| Disodium edetate | 0,50 |
| Methyl parahydroxybenzoate | 0,15 |
| Carbomer 941 (Polyacrylic acid) | 2,00 |
| Sodium hydroxide | 1,14 |
| Monohydrated citric acid | 0,60 |
| Purified water | 55,61 |
| TOTAL | 100,00 |

### Example 4.

Monohydrated citric acid, and sodium hydroxide are dissolved at room temperature in purified water receiving citrate buffer having pH equal to 5.0.

Ethanol is diluted with citrate buffer to concentration of 50%.

To the agitator tank equipped with stirrer, and heating jacket are added: azelaic acid (5%), 50% ethanol solution, citrate buffer of pH equal to 5, triethanolamine, disodium edetate, and methyl parahydroxybenzoate. The entirety is mixed at temperature of about 40°C until complete dissolution of all components.

Purified egg phosphatidylcholine is dissolved, when mixing, in propylene glycol at the temperature of about 70°C. To the so obtained solution is added by portions with continuous mixing, when deaerating, the solution of azelaic acid obtained earlier. After thorough deaeration the whole mixture is extruded once through two filters of 100 nm. To the liposome suspension is added remaining part of azelaic acid (10%). The entirety is mixed and deaerated simultaneously. Finally to the earlier obtained liposome suspension of azelaic acid is added ammonium acryloyldimethyltaurate/VP copolymer, and afterwards the entirety is thoroughly mixed and deaerated.

| *ACTIVE SUBSTANCES:* | |
|---|---|
| Azelaic acid | 15,00 |

| *EXCIPIENTS:* | |
|---|---|
| Purified egg phosphatidylcholine | 10,00 |
| Propylene glycol | 10,00 |
| Triethanolamine | 5,00 |
| Ethanol | 5,00 |
| Disodium edetate | 0,50 |
| Methyl parahydroxybenzoate | 0,15 |
| Ammonium acryloyldimethyltaurate/VP copolymer | 3,00 |
| Sodium hydroxide | 0,41 |
| Monohydrated citric acid | 1,02 |
| Purified water | 49,92 |
| TOTAL | 100,00 |

### Example 5.

Monohydrated citric acid, and sodium hydroxide are dissolved at room temperature in purified water receiving citrate buffer having pH equal to 5.0.

Ethanol is diluted with citrate buffer to concentration of 50%.

To the agitator tank equipped with stirrer, and heating jacket are added: azelaic acid (5%), 50% ethanol solution, citrate buffer of pH equal to 5, triethanolamine, disodium edetate, and methyl parahydroxybenzoate. The entirety is mixed at temperature of about 40°C until complete dissolution of all components.

1,2-dioleoyl-sn-glycero-3-phosphatidylcholine is dissolved, when mixing, in propylene glycol at the temperature of about 70°C. To the so obtained solution is added by portions with continuous mixing, when deaerating, the solution of azelaic acid obtained earlier. After thorough deaeration the whole mixture is extruded once through two filters of 100 nm. To the liposome suspension is added remaining part of azelaic acid (10%). The entirety is mixed and deaerated simultaneously. Carbomer 941 is dispergated in water. Then 30% solution of sodium hydroxide is added in order to gel the polymer. The entirety is mixed and deaerated simultaneously. To such obtained gel is added by portions the liposome suspension of azelaic acid obtained earlier. The entirety is mixed and deaerated simultaneously.

| *ACTIVE SUBSTANCES:* | |
|---|---|
| Azelaic acid | 15,00 |

| *EXCIPIENTS:* | |
|---|---|
| 1,2-Dioleoilo-*sn*-glicero-3-fosfatydylocholina | 10,00 |
| Propylene glycol | 6,67 |
| Triethanolamine | 5,00 |
| Ethanol | 3,33 |
| Disodium edetate | 0,50 |
| Methyl parahydroxybenzoate | 0,15 |
| Carbomer 941 (Polyacrylic acid) | 2,00 |
| Sodium hydroxide | 1,14 |
| Monohydrated citric acid | 0,60 |
| Purified water | 55,61 |
| TOTAL | 100,00 |

### Example 6.

Monohydrated citric acid, and sodium hydroxide are dissolved at room temperature in purified water receiving citrate buffer having pH equal to 5.0.

Ethanol is diluted with citrate buffer to concentration of 50%.

To the agitator tank equipped with stirrer, and heating jacket are added: azelaic acid (7.5%), 50% ethanol solution, citrate buffer of pH equal to 5, triethanolamine, disodium edetate, and methyl parahydroxybenzoate. The entirety is mixed at temperature of about 40°C until complete dissolution of all components.

Purified soya phosphatidylcholine is dissolved, when mixing, in propylene glycol at the temperature of about 70°C. To the so obtained solution is added by portions with continuous mixing, when deaerating, the solution of azelaic acid obtained earlier. After thorough deaeration the whole mixture is homogenised at room temperature until liposomes of about 100 nm are obtained. To the liposome suspension is added remaining part of azelaic acid (7.5%). The entirety is mixed and deaerated simultaneously. Finally to the earlier obtained liposome suspension of azelaic acid is added ammonium acryloyldimethyltaurate/VP copolymer, and afterwards the entirety is thoroughly mixed and deaerated.

| *ACTIVE SUBSTANCES:* | |
|---|---|
| Azelaic acid | 15,00 |

| *EXCIPIENTS:* | |
|---|---|
| Purified soya phosphatidylcholine | 15,00 |
| Propylene glycol | 10,00 |
| Triethanolamine | 7,50 |
| Ethanol | 5,00 |
| Disodium edetate | 0,50 |
| Methyl parahydroxybenzoate | 0,15 |
| Ammonium acryloyldimethyltaurate/VP copolymer | 1,00 |
| Sodium hydroxide | 0,36 |
| Monohydrated citric acid | 0,92 |
| Purified water | 44,57 |
| TOTAL | 100,00 |

### Example 7.

Monohydrated citric acid, and sodium hydroxide are dissolved at room temperature in purified water receiving citrate buffer having pH equal to 5.0.

Ethanol is diluted with citrate buffer to concentration of 50%.

To the agitator tank equipped with stirrer, and heating jacket are added: azelaic acid (5%), 50% ethanol solution, citrate buffer of pH equal to 5, triethanolamine, disodium edetate, and methyl parahydroxybenzoate. The entirety is mixed at temperature of about 40°C until complete dissolution of all components.

Purified soya phosphatidylcholine is dissolved, when mixing, in propylene glycol at the temperature of about 70°C. To the so obtained solution is added by portions with continuous mixing, when deaerating, the solution of azelaic acid obtained earlier. After thorough deaeration the whole mixture is homogenised at room temperature until liposomes of about 100 nm are obtained. To the liposome suspension is added remaining part of azelaic acid (10%). The entirety is mixed and deaerated simultaneously. Carbomer 941 is dispergated in water. Then 30% solution of sodium hydroxide is added in order to gel the polymer. The entirety is mixed and deaerated simultaneously. To such obtained gel is added by portions the liposome suspension of azelaic acid obtained earlier. The entirety is mixed and deaerated simultaneously.

| *ACTIVE SUBSTANCES:* | |
|---|---|
| Azelaic acid | 15,00 |

| *EXCIPIENTS:* | |
|---|---|
| Purified soya phosphatidylcholine | 10,00 |
| Propylene glycol | 6,67 |
| Triethanolamine | 5,00 |
| Ethanol | 3,33 |
| Disodium edetate | 0,50 |
| Methyl parahydroxybenzoate | 0,15 |
| Carbomer 941 (Polyacrylic acid) | 2,00 |
| Sodium hydroxide | 1,14 |
| Monohydrated citric acid | 0,60 |
| Purified water | 55,61 |
| TOTAL | 100,00 |

## Claims

1. An azelaic acid gel, **characterised in that** it consists of azelaic acid, liposomes, organic solvents, buffer, gelling agents, and may additionally consist of preservatives and antioxidants.

2. The azelaic acid gel according to claim 1, **characterised in that** the overall concentration of the azelaic acid is 15% by weight.

3. The azelaic acid gel according to claim 1 or 2, **characterised in that** liposomes are obtained from purified phosphatidylcholine, preferably from soya phosphatidylcholine or egg phosphatidylcholine.

4. The azelaic acid gel according to claim 3, **characterised in that** the overall concentration of purified phosphatidylcholine is from 10 to 15% by weight.

5. The azelaic acid gel according to claim 1 or 2, **characterised in that** liposomes are obtained from purified 1,2-dioleoyl-sn-glycero-3-phosphatidylcholine.

6. The azelaic acid gel according to claim 5, **characterised in that** the overall concentration of 1,2-dioleoyl-sn-glycero-3-phosphatidylcholine is from 10 to 15% by weight.

7. The azelaic acid gel according to any of the preceding claims, **characterised in that** it comprises monolayer liposomes.

8. The azelaic acid gel according to any of the claims from 1 to 6, **characterised in that** it comprises multilayer liposomes.

9. The azelaic acid gel according to any of the claims from 1 to 6, **characterised in that** it comprises mixture of monolayer liposomes and multilayer liposomes.

10. The azelaic acid gel according to any of the preceding claims, **characterised in that** azelaic acid is not bound to liposomes.

11. The azelaic acid gel according to any of the claims from1 to 9, **characterised in that** azelaic acid is bound to liposomes.

12. The azelaic acid gel according to any of the claims from1 to 9, **characterised in that** azelaic acid is both bound and not bound to liposomes.

13. The azelaic acid gel according to any of the preceding claims, **characterised in that** the organic solvents are propylene glycol, ethanol and triethanolamine.

14. The azelaic acid gel according to claim 13, **characterised in that** the overall concentration of propylene glycol is from 6 to 10% by weight.

15. The azelaic acid gel according to claim 13 or 14, **characterised in that** the overall concentration of ethanol is from 3 to 5% by weight.

16. The azelaic acid gel according to claim 13 or 14 or 15, **characterised in that** the overall concentration of triethanolamine is from 7 to 7.5% by weight.

17. The azelaic acid gel according to any of the preceding claims, **characterised in that** the buffer is citrate buffer.

18. The azelaic acid gel according to claim 17, **characterised in that** the pH of buffer citrate is between 4.5 and 5.5.

19. The azelaic acid gel according to any of the preceding claims, **characterised in that** the antioxidant is disodium edetate.

20. The azelaic acid gel according to claim 19, **characterised in that** the overall concentration of disodium edetate is 0.5% by weight.

21. The azelaic acid gel according to any of the preceding claims, **characterised in that** the preservative is methyl parahydroxybenzoate.

22. The azelaic acid gel according to claim 21, **characterised in that** the overall concentration of methyl parahydroxybenzoate is 0.15% by weight.

23. The azelaic acid gel according to any of the preceding claims, **characterised in that** the gelling agent is polyacrylic acid.

24. The azelaic acid gel according to claim 23, **characterised in that** the overall concentration of polyacrylic acid is from 1 to 3% by weight.

25. The azelaic acid gel according to any of the claims from 1 to 22, **characterised in that** the gelling agent is ammonium acryloyldimethyltaurate/VP copolymer.

26. The azelaic acid gel according to claim 25, **characterised in that** the overall concentration of ammonium acryloyldimethyltaurate/VP copolymer is from 1 to 3% by weight.

27. A method of obtaining the azelaic acid gel consisting of azelaic acid, liposomes, organic solvents, buffer, gelling agents, and preservatives and/or antioxidants, **characterised in that** purified phosphatidylcholine or 1,2-dioleoyl-sn-glycero-3- phosphatidylcholine is dissolved in propylene glycol, and then mixed with aqueous phase comprising buffer, remaining organic solvents, antioxidant, preservative and azelaic acid to obtain a suspension of liposomes, which is then mixed with remaining part of azelaic acic and gelling substance.

28. A method of obtaining the azelaic acid gel according to claim 27, **characterised in that** the suspension of liposomes is obtained by extrusion.

29. A method of obtaining the azelaic acid gel according to claim 27, **characterised in that** the suspension of liposomes is obtained by homogenisation.
